# EUROPEAN PATENT APPLICATION

(11) **EP 3 391 895 A1**
(43) Date of publication of application: **24.10.2018**
(21) Application number: 18167750.1
(22) Date of filing: 17.04.2018
(51) Int. Cl.: A61K 36/9066, A61K 31/185, A61K 31/198, A61K 33/10, A61P 39/02

(54) **COMPOSITIONS USEFUL FOR THE TREATMENT OF SYMPTOMS ASSOCIATED WITH ALCOHOL ABUSE**

(30) Priority: 18.04.2017 IT 201700042325
(71) Applicant: Ecillax S.r.l., 22100 Como (IT)
(72) Inventor: ROSSI, Francesco, 22100 COMO (IT)
(74) Representative: Bianchetti Bracco Minoja S.r.l.

(57) **Abstract**

Disclosed are compositions useful for the treatment of symptoms associated with alcohol abuse, comprising *Phyllanthus spp* extract, preferably *Phyllanthus emblica* or *niruri* extract, *Curcuma Longa* extract or root, taurine, N-acetylcysteine and calcium carbonate.

## Description

The present invention relates to compositions useful for the treatment of symptoms associated with alcohol abuse, comprising *Phyllanthus spp* extract, preferably *Phyllanthus emblica* or *niruri* extract, extract or root of *Curcuma Longa,* taurine, N-acetylcysteine and calcium carbonate.

### Prior art

Alcohol abuse is a major problem in our society, causing an average of 3.3 million deaths a year worldwide. Excessive alcohol consumption can lead to premature death (it is the third most important risk factor after smoking and hypertension), and to the development of numerous disorders affecting various organs and tissues, such as cirrhosis of the liver, liver cancer, pancreatitis, oesophagitis, steatosis, infertility, impotence, hormonal alterations, cardiomyopathy and stroke. Excessive alcohol consumption can also cause damage to the central and peripheral nervous systems, such as cerebral atrophy and polyneuritis.

Chronic alcohol abuse produces effects similar to those of other psychoactive substances, including illegal drugs, leading to mental and physical dependence, addiction and other behavioural disorders which have significant repercussions for the individual, including in terms of social interactions.

Acute sporadic alcohol consumption, known as "binge drinking", is widespread among the younger generation nowadays, especially at weekends. This trend causes specific symptoms for the individual such as nausea, headache, development of gastric acidity, dizziness, fatigue, muscle pains and tachycardia. These disorders also continue in the days following the episode of abuse, and are characteristic of the "hangover" state.

The symptoms of acute alcohol poisoning are correlated with a high blood concentration of acetaldehyde (a metabolite of ethanol), induced by an overload of the enzyme acetaldehyde dehydrogenase, which converts the molecule to acetate.

Excessive alcohol consumption, even if sporadic, is known to be associated with a modification in the body's redox balance, which leads to an increase in pro-oxidant species, a condition that gives rise to the state of oxidative stress. Many scientific studies have demonstrated that ethanol increases lipid peroxidation and protein modification; however, it is still unclear whether said changes are the cause or result of the tissue lesions caused by alcohol. Alcohol abuse, even if occasional, induces a significant increase in the synthesis of various cytokines, such as interleukin-10 (IL-10), interleukin-12 (IL-12) and interferon-γ (IFN-γ), which are involved in the immune defenses, and whose physiological levels remain modified during the hangover period. This suggests that the immune system is also altered by excessive, albeit sporadic alcohol consumption.

The hangover, as well as having adverse effects on the health, also has an adverse impact on the individual's social interactions and financial situation.

Some observational studies have demonstrated that hangovers
1) contribute to absenteeism,
2) reduce working performance,
3) reduce memory performance,
4) can seriously prejudice the activities of driving vehicles or using machinery, thus increasing the risk of road accidents and accidents in the workplace.

The hangover consists of a feeling of malaise and objective dysfunctional aspects after an episode of drinking a significant amount of alcohol, higher than that normally consumed by a given person. This unwell feeling leads to reduced performance at work and often gives rise to sick leave, involving significant costs. The malaise, and therefore the frequency of sick leave, is greater in first-time drinkers, former drinkers and heavy drinkers than in individuals who regularly drink a moderate amount of alcohol. The effects take the form of poor memory and visuospatial awareness, which can give rise to accidents in the workplace. Other symptoms include dizziness, nausea and difficulty in concentrating. This set of symptoms seems to be due to the effects of alcohol and its main intermediate metabolite, acetaldehyde. The effects can be direct or mediated by the actions of alcohol and acetaldehyde on the operation of the nervous, endocrine and immune systems, or can be the consequence of dehydration and sleep disorders due to consumption of massive doses of alcohol.

Various pharmacological remedies have been proposed which are designed to support the liver function in the metabolism of alcohol, to replenish water, minerals and vitamins depleted by alcohol, limit nausea and gastric disorders, and support the mental functions. Examples of compositions for the treatment of hangover are described, for example, in KR101476092, EP 2 792 364, WO 2016/142580, US 9 101 657, US 9 474 802. The most widely studied active ingredients comprise phytotherapeutic extracts (such as extracts of *Curcuma*, *Zingiber officinalis*, *Ganoderma lucidum, Cynara spp*., *Lycium barbarum*, *Malpighia pinicifolia*, *Opuntia ficus*, *Quercus ilex* and *Laurus nobilis*), vitamins (vitamin C, vitamin B6, vitamin B12, thiamine and riboflavin), folic acid, glutamic acid, N-acetylcysteine, alpha-lipoic acid, caffeine, taurine, dihydromyricetin, acetylsalicylic acid, trace elements and calcium.

For instance, KR 100 392 876 discloses compositions comprising an extract of *Curcuma Longa* (turmeric); DE 10 2011 013224 discloses compositions comprising taurine, N-acetylcysteine and calcium; CN 104 001 135 discloses compositions comprising extract of *Phyllantus spp.* and taurine; Taranukhin Andrey Get al.:, Journal of Biomedical Science, DO, vol. 17, no. Suppl 1, 24 August 2010, page S12, discloses compositions comprising taurine; US 9 101 657 discloses compositions comprising an extract or root of *Curcuma Longa* ; US 2015/202181 discloses compositions comprising N-acetylcysteine; KR 2009 0078662 discloses compositions comprising calcium; Krithika Rajesh et al:, Toxicology and Industrial Health, vol. 32, no. 5, May 2016, pages 953-960 disclose compositions comprising an extract of *Phyllantus spp*.; JP 2001 238650 discloses compositions comprising turmeric, *Panax ginseng* and *Ganoderma lucidum* and US 2007/065456 discloses compositions comprising curcumin (extract of *Curcuma Longa),* taurine, *Phyllanthus* extract and thiamine.

None of said compositions comprise antacids.

The presence of an antacid would be desirable since alcohol induces gastric lesions and facilitates the development of gastroesophageal reflux disease increasing gastrin levels and acid secretion.

The conventional antacids such as aluminum and magnesium hydroxide are however known to adversely affect the stability of most active ingredients contained in the known compositions for the treatment of hangover, particularly those containing plant extracts and N-acetylcysteine. There is accordingly the need for compositions including an antacid combined with phytotherapeutic extracts and other ingredients so as to secure an effective and safe treatment of hangover.

### Description of the invention

It has been found that calcium carbonate, while securing an effective antacid efficacy, does not substantially impair the stability of phytotherapeutic extracts and of other ingredients suitably selected for their beneficial effects on the clinical symptoms following alcohol abuse.

The invention provides a combination of *Phyllanthus spp* extract, preferably *Phyllanthus emblica* or *niruri* extract, *Curcuma Longa* extract or root, taurine, N-acetylcysteine and calcium carbonate, optionally mixed with suitable carriers and excipients, and optionally with other active ingredients.

*Phyllanthus emblica* L. is a fruit widely used for its anti-inflammatory, antipyretic, tonic, adaptogenic, antimicrobial, immunomodulating and antioxidant properties, due to the numerous bioactive molecules contained in its extract, especially vitamin C and tannins.

The components of *P. emblica* perform a protective function towards the liver cells, protecting them against the damage caused by the toxic molecules produced by oxidative stress and inflammation.

*P. emblica* also acts in the brain, wherein it has a protective effect on the nerve cells due to its action against the mitochondrial dysfunction induced by alcohol.

The *Phyllanthus emblica* dosage unit in the compositions according to the invention ranges between 50 and 200 mg.

*Curcuma Longa* L. is a plant rich in bioactive molecules that possess numerous health-giving and therapeutic properties, the most widely investigated of which are its anti-inflammatory, gastroprotective, liver-protective and antitumor properties. Curcumin, which is the main ingredient in turmeric extracts, presents an antioxidant activity comparable with that of vitamin C and vitamin E. *C. longa,* due to the antioxidant activity of its ingredients, performs an important liver-protecting function, as demonstrated by numerous studies present in the scientific literature. *C. longa* also has anti-ulcer and gastroprotective effects. It has been demonstrated that pectic polysaccharides isolated from *C. longa* regulate the activity of the H+/K+ ATPase proton pump in the stomach, reducing or even inhibiting the growth and adherence of *H. pylori* to the gastric mucosa.

The dosage unit of *Curcuma Longa* extract or root in the compositions according to the invention ranges between 50 and 200 mg.

N-Acetylcysteine (NAC) is the direct precursor of glutathione, an endogenous antioxidant molecule. NAC, due to its ability to replenish the intracellular glutathione levels in the event of deficiency, is widely used as a diet supplement in the treatment of metabolic disorders, lung diseases and liver and neurological toxicity.

An excessive ethanol intake leads to the development of liver damage, due to the increased ROS concentration. Hepatic metabolism of ethanol produces acetaldehyde, a cytotoxic metabolite, the action of which is neutralised by the bond with glutathione. This process leads to a reduction in the intracellular glutathione concentration, and NAC administration therefore counteracts said reduction following ethanol consumption. NAC also has gastroprotective effects.

The N-acetylcysteine dosage unit in the compositions according to the invention ranges between 100 and 400 mg.

Taurine has performance-enhancing, antioxidant and anti-inflammatory properties. *In vitro* studies have demonstrated that taurine deficiency damages the cellular respiratory chain, leading to a reduction in the uptake of long-chain fatty acids in the mitochondria and a consequent reduction in ATP production.

*In vitro* and *in vivo* studies have demonstrated that taurine has a neuroprotective activity against the toxicity induced by alcohol and other toxic substances, protecting the brain cells against oxidative stress. In particular, taurine has a neuroprotective effect against toxicity induced by glutamate, an excitotoxic amino acid. Various *in vitro* studies have demonstrated the anti-inflammatory activity of taurine. The taurine dosage unit in the compositions according to the invention ranges between 100 and 500 mg, while the calcium carbonate dosage unit ranges between 100 and 1000 mg.

The compositions according to the invention can also contain one or more ingredients selected from extracts of *Zingiber officinalis*, *Panax ginseng, Ganoderma lucidum* and thiamine. Suitable forms of administration comprise capsules, tablets, powders, granulates and gels. The compositions can be administered one to three times a day.

The invention is illustrated in greater detail in the following examples.

### EXAMPLE 1

| | | dose |
|---|---|---|
| *Phyllanthus emblica* (dried fruit extract) | mg | 100 |
| Taurine | mg | 300 |
| *Curcuma Longa* root | mg | 100 |
| Acetyl cysteine | mg | 300 |
| Calcium carbonate | mg | 500 |

### EXAMPLE 2

| | | | |
|---|---|---|---|
| *Zingiber officinale* powdered root | | mg | 50 |
| *Curcuma Longa* root | | mg | 100 |
| *Panax ginseng* (5% ginsenosides) | | mg | 25 |
| Taurine | | mg | 300 |
| *Ganoderma lucidum* | | mg | 2 |
| *Phyllanthus emblica* (dried fruit extract) | | mg | 100 |
| Thiamine (as thiamine HCl) | | mg | 25 |
| Acetylcysteine | | mg | 300 |
| Calcium carbonate | | mg | 500 |
| | | | |
| Thickener | Xanthan gum | | |
| Sweetener | Sucralose, fructose | | |
| Flavouring agents | | | |
| Preservatives: | Sodium benzoate, potassium sorbate | | |

### EXAMPLE 3

Aqueous solutions of *Curcuma Longa* extract, *Phyllantus emblica* extract and N-acetyl cysteine in the presence of either calcium carbonate or of magnesium hydroxide have been analyzed by means of reverse phase HPLC coupled to diode-array detector and to a mass spectrometer (RP-HPLC-DAD-ESI-MSn) in order to evaluate the chemical stability of the main active ingredients of the extracts and of N-acetylcysteine in the presence of antacids. In particular, the following solutions were prepared:
1) *Curcuma L.* extract (50 mg) + CaCO₃ (500 mg) / 10 ml H₂O
*2) Curcuma L.* extract (50 mg) + Mg(OH)₂/ 10 ml H₂O
*3)P. emblica* extract (200 mg) + CaCO₃ (1000 mg)/50 ml water/methanol 30:70
*4) P. emblica* extract (200 mg) + Mg(OH)₂ (1000 mg) 50 ml water/methanol 30:70
5) NAC (300 mg) + CaCO₃ (500 mg)/10 ml H₂O
6) NAC (300 mg) + Mg(OH)₂ (500 mg)/10 ml H₂O.

The content of curcumin in the first two solutions, the content of gallic acid in solutions 3) and 4) and the content of NAC in solutions 5) and 6) was determined at different time intervals substantially according to the method reported by Kumar S. et al. in Journal of Pharmaceutical Analysis. 2017, 7: 241-222. The results are reported in the following tables 1-3.

**Table 1: area of peaks corresponding to curcumin content in Curcuma L. solutions.**

| **Time** | | **Curcuma + CaCO₃** | **Curcuma + Mg(OH)₂** |
|---|---|---|---|
| Hours | Days | | |
| 0 | | 6659.58 | 8838.45 |
| 7 | | 3775.29 | 2074.35 |
| 14 | | 3068.88 | 839.714 |
| 21 | | 2601.34 | 476.352 |
| 72 | 3 | 1489.42 | 246.3538 |
| 96 | 4 | 1313.53 | 221.6467 |
| 120 | 5 | 1192.44 | 205.6069 |
| 144 | 6 | 1028.94 | 198.3625 |
| 168 | 7 | 980.451 | 177.9218 |

**Table 2: area of peaks corresponding to gallic acid content in P. emblica solutions.**

| **Time** | | ***P. emblica* (gallic acid) + CaCO₃** | ***P. emblica* (gallic acid) + M(OH)₂** |
|---|---|---|---|
| Hours | Days | | |
| 0 | | 93277284 | 61728601 |
| 7 | | 85724377 | 48815053 |
| 14 | | 81955603 | 49652304 |
| 21 | | 72277352 | 49055079 |
| 72 | 3 | 70104859 | 49773749 |
| 96 | 4 | 70054334 | 49222997 |
| 120 | 5 | 68933391 | 49520819 |
| 144 | 6 | 68374381 | 48807141 |
| 168 | 7 | 68077103 | 48241175 |
| 192 | 8 | 68291103 | 48970862 |
| 216 | 9 | 68077103 | 48241175 |

**Table 3. Area of peaks of NAC**

| **Time** | | **NAC + CaCO₃** | **NAC + Mg(OH)₂** |
|---|---|---|---|
| Hours | days | | |
| 24 | 1 | 42558208 | 29420562 |
| 48 | 2 | 41625724 | 29739150 |
| 72 | 3 | 40360431 | 28810967 |

The results reported in Tables 1-3 show that the solutions containing calcium carbonate turn out to be significantly more stable than the solutions containing magnesium hydroxide.

## Claims

1. An oral composition comprising extract of *Phyllantus spp.,* extract or root of *Curcuma Longa,* taurine, N-acetylcysteine and calcium carbonate.

2. A composition according to claim 1 wherein the dosage unit of *Phyllantus spp.* ranges from 50 to 200 mg.

3. A composition according to claim 1 or 2 wherein the extract of *Phyllantus spp.* is an extract of *Phyllantus emblica* or *Phyllantus niruri.*

4. A composition according to claim 1, 2 or 3 wherein the dosage unit of extract or root of *Curcuma Longa* ranges from 50 to 200 mg.

5. A composition according to any one of claims 1-4 wherein the dosage unit of N-acetylcysteine ranges from 100 to 400 mg.

6. A composition according to any one of claims 1-5 wherein the dosage unit of calcium carbonate ranges from 100 to 1000 mg.

7. A composition according to any one of claims 1-6 wherein the dosage unit of taurine ranges from 100 to 500 mg.

8. A composition according to one or more of claims 1-7 further comprising one or more ingredients selected from extracts of *Zingiber officinalis*, *Panax ginseng, Ganoderma lucidum* and thiamine.

9. A composition according to one or more of claims 1-8 in the form of capsules, tablets, powders, granulates or gels.

10. A composition according to claims 1-9 for use in the treatment of hangover.
